# EUROPEAN PATENT SPECIFICATION

(11) **EP 1 943 267 B1**
(45) Date of publication and mention of the grant of the patent: **16.03.2011**
(21) Application number: 06776747.5
(22) Date of filing: 10.08.2006
(51) Int. Cl.: C07K 5/06, A61K 38/05

(54) **A PROCESS FOR THE PREPARATION OF PERINDOPRIL ERBUMINE**
VERFAHREN ZUR HERSTELLUNG VON PERINDOPRILERBUMIN
PROCEDE DE PREPARATION DE PERINDOPRIL ERBUMINE

(30) Priority: 12.08.2005 SI 200500232
(43) Date of publication of application: 16.07.2008
(73) Proprietor: LEK Pharmaceuticals d.d., 1526 Ljubljana (SI)
(72) Inventor: HAM, Zoran, 1420 Trbovlje (SI); FURLAN, Borut, 1000 Ljubljana (SI)
(86) International application number: PCT/EP2006/007926
(87) International publication number: WO 2007/020012

(56) References cited:
- EP-A- 1 321 471
- EP-A- 1 333 026
- WO-A-01/83439
- WO-A-01/87836
- GB-A- 2 395 195
- US-A1- 2005 059 609
- US-A1- 2005 171 165

## Description

The present invention relates to a new process for the preparation of pure perindopril erbumine. The present invention also relates to a new process for the preparation of crystalline form D of perindopril erbumine.

Perindopril and its pharmaceutically acceptable salts are known as angiotensin converting enzyme inhibitors and are used in the treatment of cardiovascular diseases, especially in the treatment of hypertension and heart failure. Perindopril is chemically known as (2S,3aS,7aS)-((2-(1-(ethoxycarbonyl)-(S)-butylamino)-(S)-propionyl)octahydro-indole-2-carboxylic acid and can be represented by formula (I).

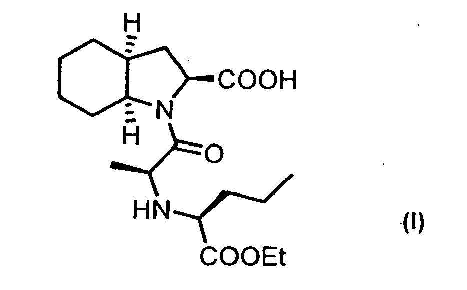

Perindopril was first disclosed in EP 0049658 B1 and US 4,508,729 as optically pure S,S,S,S,S isomer and in the form of sodium salt. Numerous later patents and patent applications, such as EP 0308341 B1, EP 1279665 A1, EP 1333026 A1, WO 2004/099236 describe various processes for the preparation of perindopril.

tert-Butylamine salt of perindopril, known as perindopril erbumine, which is widely used in pharmaceutical products, was first disclosed in EP 0308341 B1. Perindopril erbumine may be obtained in different crystalline forms depending on crystallization conditions, e.g. solvent system, perindopril erbumine concentration and cooling kinetics. EP 1296947 B1 discloses crystallization of perindopril erbumine crystalline form a from ethyl acetate, EP 1294689 A discloses crystallization of perindopril erbumine crystalline form β from dichloromethane or ethyl acetate, EP 1296948 B1 discloses crystallization of perindopril erbumine crystalline form γ from chloroform, and WO 2004/113293 discloses crystallization of perindopril erbumine crystalline form δ and crystalline form ε. Crystalline form ε is obtained by crystallization from tert-butyl methyl ether containing 1.5 to 2.5 % (vol/vol) of water whereas crystalline form δ is obtained from form ε by azeotropic distillation.

US2005/059609 A1 describes the crystallization of perindopril erbumine in ethyl acetate. GB2395195 describes the crystallization of perindopril erbumine in ethyl acetate containing 0.5% (vol/vol) water, so as to form perindropril erbumine monohydrate.

EP 0308341 B1 describes an industrial process for the preparation of perindopril by coupling of protected (2S,3aS,7aS)-2-octahydroindole-2-carboxylic acid with N-[(S)-1-carbethoxybutyl]-(S)-alanine in the presence of dicyclohexylcarbodiimide and 1-hydroxybenzotriazole. Perindopril erbumine is obtained from crude perindopril by crystallization after adding of tert-butylamine. The drawback of this process is the formation of by-products derivable from dicyclohexylcarbodiimide, which are difficult to remove. For this reason, additional purification steps are needed to obtain pure perindopril and/or pure perindopril erbumine.

In addition to the by-products formed during the process of the preparation, also degradation products of perindopril are present as impurities in crude perindopril. Perindopril and its salts are chemically highly sensitive compounds and are susceptible to degradation via a) isomerisation at some chiral centres, b) hydrolysis of the side-chain ester group and/or c) intramolecular cyclization to form diketopiperazines. Two most critical diketopiperazines are (R)-ethyl 2-((3S,5aS,9aS,10aS)-3-methyl-1,4-dioxo-decahydropyrazino[1,2-a]indol-2(1H)-yl)pentanoate noted as diketopiperazine I and 2-((3S,5aS,9aS,10aR)-3-methyl-1,4-dioxo-decahydropyrazino[1,2-a]indol-2(1H)-yl)pentanoic acid noted as diketopiperazine II (formulas as depicted below), also indicated in European Pharmacopoea 5.1 as impurities F and C, respectively.

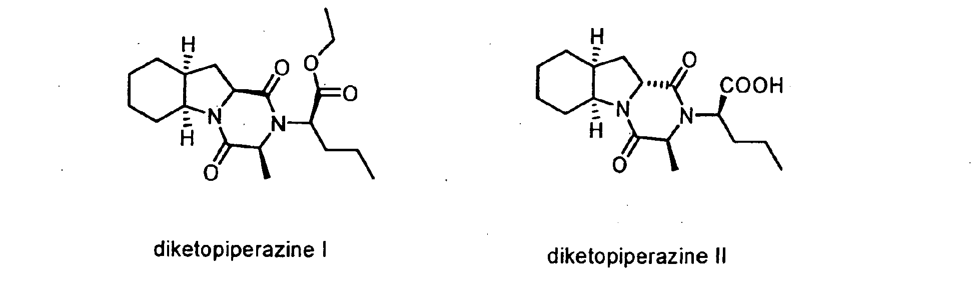

WO 01/58868 describes an improved process for the preparation of perindopril from (2S,3aS,7aS)-octahydroindole-2-carboxylic acid benzyl ester para-toluenesulfonate and N-[(S)-ethoxycarbonyl-1-butyl]-(S)-alanine in the presence of dicylohexylcarbodiimide, 1-hydroxybenzotriazole and optionally triethylamine. The formation of by-products derivable from dicyclohexylcarbodiimide is diminished by strict control of amounts of reagents used in the process. However, the described process per se does not solve the problem of other impurities.

The problems of impurities derivable from dicyclohexylcarbodiimide is completely solved by processes for the preparation of perindopril that avoid use of dicyclohexylcarbodiimide. For example, such processes are described in patent applications EP 1279665 A1 and EP 1333026 A1. However, perindopril erbumine obtained by reaction of tert-butylamine with crude perindopril prepared according to the processes disclosed in these patent applications has to be additionally purified by different purification methods, such as thermal recrystallization or treatment with charcoal.

One can also mention WO 2004/046172 describing a process for the preparation of high pure perindopril erbumine, wherein a protected precursor of perindopril is deprotected in the presence of a base, e.g. tert-butylamine, to obtain perindopril salt, e.g. perindopril erbumine, directly without isolation of crude perindopril. A formation of diketopipirazine impurities during the manufacturing process is minimized due to short reaction time. However, other impurities have to be removed from obtained perindopril erbumine by additional crystallization step.

WO 2005/019173 discloses a process for the preparation of pure perindopril erbumine from crude perindopril by extracting aqueous solution of crude perindopril or its salt with suitable organic solvent at pH from 4.0 to 6.5, followed by separating of organic layer and preparing perindopril erbumine by adding tert-butylamine. The drawback of this process is a high number of steps that may result in low yield.

There is a continuing need for developing simple and effective process for the preparation of pure perindopril erbumine from crude perindopril, which does not require additional purification of obtained perindopril erbumine, and is applicable at the industrial scale.

The present invention provides an improved process for the preparation of pure perindopril erbumine from crude perindopril, said improved process being especially effective in removing diketopiperazine impurities. Furthermore, said process is simple and it is applicable on an industrial scale.

A first object of the present invention is related to a process for the preparation of crystalline perindopril erbumine comprising the steps of:
(a) providing a solution of crude perindopril in wet aliphatic ester containing from 1% (vol/vol) to 6% (vol/vol) water or in a mixture of wet aliphatic esters containing from 1% (vol/vol) to 6% (vol/vol) water,
(b) adding tert-butylamine to the said solution,
(c) crystallizing perindopril erbumine, and
(d) isolating crystalline perindopril erbumine.

The process of the present invention allows to obtain a pure perindopril erbumine containing less than 0.20 % (w/w) of diketopiperazine impurities, preferably containing less than 0.10 % (w/w) of diketopiperazine impurities.

"Wet aliphatic ester" in the present invention means the aliphatic ester enriched with from 1 % (vol/vol) to 6% (vol/vol) of water, more preferably from 2% (vol/vol) to 4% (vol/vol) of water, most preferably from 2% (vol/vol) to 3% (vol/vol) of water.

In step (a), wet aliphatic ester is preferably selected from the group consisting of wet C₁-C₄ alkyl esters of C₁-C₄ aliphatic carboxylic acids. Preferably wet C₁-C₄ alkyl esters of C₁-C₄ aliphatic carboxylic acids include wet ethyl acetate, wet isopropyl acetate, wet butyl acetate and wet ethyl propionate. More preferably the wet aliphatic ester used in step (a) is wet ethyl acetate. The wet ethyl acetate used in step (a) contains from 1% (vol/vol) to 6% (vol/vol) of water, more preferably from 2% (vol/vol) to 4% (vol/vol) of water, most preferably from 2% (vol/vol) to 3% (vol/vol) of water.

The saturation of ethyl acetate can be executed by shaking it with water in a separation funnel with further separating of water phase. In order to avoid residual drops of water, ethyl acetate phase was cooled, preferably to temperature from -20°C to -10 °C, and carefully decanted from drops of water. Such ethyl acetate contains water in concentration slightly below saturation at room temperature and is one of the preferred wet aliphatic esters usable in step (a).

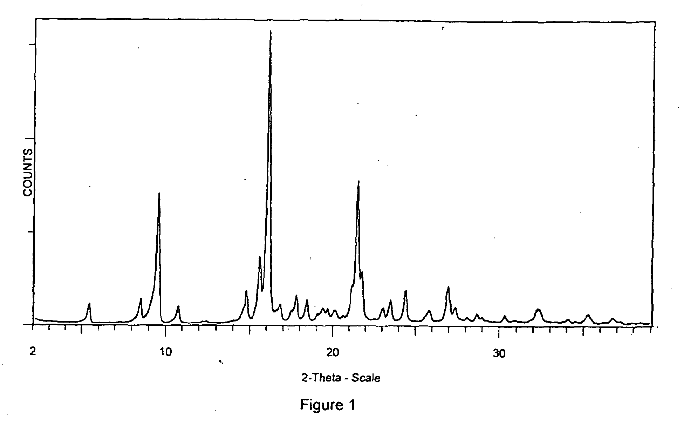

It has surprisingly been found that when the crystallization of perindopril erbumine is carried out in wet aliphatic ester or in a mixture of wet aliphatic esters according to the process of the present invention, the obtained perindopril erbumine crystalline form does not correspond to the crystalline forms known from the prior art, but to a new crystalline form of perindopril erbumine having a different X-ray powder diffraction pattern. New crystalline form of perindopril erbumine, named form D, has a powder x-ray diffraction pattern comprising the following characteristic reflection angles 2θ: 5.3±0.2°, 10.7±0.2°, 16.0±0.2°, 24.4±0.2° and 26.9±0.2°. New crystalline form D has a powder x-ray diffraction pattern as depicted in Figure 1 having the following characteristic 2θ angles:

| Angle 2θ (°) | Relative intensity (%) |
|---|---|
| 5.3 | 4.7 |
| 8.4 | 7.0 |
| 9.4 | 34.4 |
| 10.7 | 5.0 |
| 14.7 | 15.7 |
| 15.5 | 33.3 |
| 16.0 | 100.0 |
| 16.7 | 6.6 |
| 17.7 | 9.2 |
| 18.3 | 10.2 |
| 21.1 | 22.1 |
| 21.5 | 59.3 |
| 21.7 | 25.6 |
| 23.0 | 6.0 |
| 23.5 | 9.0 |
| 24.4 | 12.7 |
| 25.7 | 6.9 |
| 26.9 | 18.1 |
| 27.3 | 6.7 |
| 28.1 | 2.8 |

Another embodiment object of the present invention is related to a process for the preparation of perindopril erbumine crystalline form D comprising the steps of:
   (a) providing a solution of crude perindopril in wet aliphatic ester containing from 1% (vol/vol) to 6% (vol/vol) water or in a mixture of wet aliphatic esters containing from 1% (vol /vol) to 6% (vol/vol) water
   (b) adding tert-butylamine to the said solution,
   (c) crystallizing perindopril erbumine crystalline form D, and
   (d) isoiaiing perindoprii erbumine crystalline form D.

The process of the present invention allows to obtain a pure perindopril erbumine crystalline form D containing less than 0.20 % (w/w) of diketopiperazine impurities, preferably containing less than 0.10 % (w/w) of diketopiperazine impurities.

In step (a), said solution of crude perindopril in wet aliphatic ester or a mixture of wet aliphatic esters may be provided by dissolving crude perindopril in wet aliphatic ester or a mixture of wet aliphatic esters, optionally followed by removing of insoluble impurities by filtration. In another option, in step (a) also a solution, or a suspension of crude perindopril may be used for providing a solution of crude perindopril in wet aliphatic ester or in a mixture of wet aliphatic esters. Alternatively a solution of crude perindopril may be provided by an appropriate chemical reaction.

In a specific process according to the invention step (a) comprises the following sub-steps of:
(a1) dissolving crude perindopril in wet aliphatic ester or a mixture of wet aliphatic esters containing from 1% (vol/vol) to 6% (vol/vol) of water, and
(a2) removing of insoluble impurities by filtration.

In a specific process according to the invention step (a) comprises the following sub-steps of:
(a1') dissolving crude perindopril in wet ethyl acetate containing from 2% (vol/vol) to 4% (vol/vol) of water, and
(a2') removing of insoluble impurities by filtration.

In step (b), tert-butylamine is added preferably at temperature between -20 °C and boiling point of tert-butylamine, more preferably at temperature from 20 °C to 40 °C.

In a specific process according to the invention step (c) comprises the following sub-steps of:
(c1) heating the mixture obtained from step (b) up to the boiling point of the used aliphatic ester or the mixture of aliphatic esters,
(c2) filtration of the obtained boiling solution, and
(c3) cooling the obtained filtrate below 40 °C to obtain crystalline perindopril erbumine, preferably perindopril erbumine crystalline form D.

In sub-step (c3), the filtrate is preferably cooled below 20 °C, more preferably to temperature from -10 °C to 0 °C.

In a specific process according to the invention step (c) comprises the following sub-steps of:
(c1') heating the mixture obtained from step (b) up to the boiling point of the used aliphatic ester or the mixture of aliphatic esters,
(c2') filtration of the obtained boiling solution, and
(c3') cooling the obtained filtrate below 20 °C to obtain crystalline perindopril erbumine, preferably perindopril erbumine crystalline form D.

In a specific process according to the invention, step (c) comprises the following sub-steps of:
(c1") heating the mixture obtained from step (b) up to the boiling point of the used aliphatic ester or the mixture of aliphatic esters,
(c2") filtration of the obtained boiling solution, and
(c3") cooling the obtained filtrate to temperature from -10 °C to 0 °C to obtain crystalline perindopril erbumine, preferably perindopril erbumine crystalline form D.

Preferably, a mixture containing perindopril erbumine crystalline form D obtained after cooling according to sub-step (c3), (c3') or (c3") is left without agitation or stirring for about 15 to about 60 minutes, preferably for about 15 to about 45 minutes, more preferably for about 30 minutes, before the isolation of perindopril erbumine crystalline form D (step (d)) is carried out.

In a specific process according to the invention, step (d) comprises the following sub-steps:
(d1) isolation of crystalline perindopril erbumine, preferably perindopril erbumine crystalline form D, obtained from step (c) by filtration or centrifugation, preferably by filtration, and
(d2) drying of crystalline perindopril erbumine, preferably perindopril erbumine crystalline form D.

The filtration of crystalline perindopril erbumine, preferably perindopril erbumine crystalline form D, in sub-step (d1) is preferably performed at temperature below 0 °C, more preferably at temperature from -20 °C to -10 °C in order to guarantee good yield and quality of obtained crystalline perindopril erbumine.

The preferred temperature of drying performed in sub-step (d2) is from 25 °C to 50 °C, more preferred from 30 °C to 40 °C. Preferably crystalline perindopril erbumine is dried to the constant weight.

In a specific process according to the invention step (d) comprises the following sub-steps:
(d1') isolation of crystalline perindopril erbumine, preferably perindopril erbumine crystalline form D, obtained from step (c) by filtration at temperature from -20 °C to -10 °C, and
(d2') drying of crystalline perindopril erbumine, preferably perindopril erbumine crystalline form D, at temperature from 30 °C to 40 °C.

A preferred process according to the invention comprises the following sub-steps of:
(a1) dissolving crude perindopril in wet aliphatic ester or in a mixture of wet aliphatic esters, containing from 1% (vol/vol) to 6% (vol/vol) of water,
(a2) removing of insoluble impurities by filtration,
(b) adding tert-butylamine to the solution obtained from step (a2), preferably at temperature between -20 °C and boiling point of tert-butylamine,
(c1) heating the mixture obtained from step (b) up to the boiling point of the used aliphatic ester or the mixture of wet aliphatic esters,
(c2) filtration of the obtained boiling solution,
(c3) cooling the obtained filtrate below 40 °C to obtain crystalline perindopril erbumine,
(d1) isolation of crystalline perindopril erbumine obtained from step (c3) by filtration or centrifugation, preferably by filtration, and
(d2) drying of perindopril erbumine crystalline form D.

A preferred process for the preparation of perindopril erbumine crystalline form D according to the invention comprises the following sub-steps of:
(a1') dissolving crude perindopril in wet ethyl acetate containing from 1% (vol/vol) to 6% (vol/vol) water and
(a2') removing of insoluble impurities by filtration,
(b') adding tert-butylamine to the solution obtained from step (a2) at temperature from 20 °C to 40 °C,
(c1") heating the mixture obtained from step (b') up to the boiling point of ethyl acetate,
(c2") filtration of the obtained boiling solution,
(c3") cooling the obtained filtrate to temperature from -10 °C to 0 °C to obtain perindopril erbumine crystalline form D,
(d1') isolation of perindopril erbumine crystalline form D obtained from step (c3") by filtration at temperature from -20 °C to -10 °C, and
(d2') drying of perindopril erbumine crystalline form D at temperature from 30 °C to 40 °C.

A more preferred process for the preparation of perindopril erbumine crystalline form D according to the invention comprises the following sub-steps of:
(a1') dissolving crude perindopril in wet ethyl acetate containing from 1% (vol/vol) to 6% (vol/vol) water and (a2') removing of insoluble impurities by filtration,
(b') adding tert-butylamine to the solution obtained from step (a2) at temperature from 20 °C to 40 °C,
(c1") heating the mixture obtained from step (b') up to the boiling point of ethyl acetate,
(c2") filtration of the obtained boiling solution,
(c3") cooling the obtained filtrate to temperature from -10 °C to 0 °C to obtain perindopril erbumine crystalline form D,
(c4") left a mixture obtained from step (c3") without agitation for 15 to 60 minutes,
(d1') isolation of perindopril erbumine crystalline form D obtained from step (c4") by filtration at temperature from -20 °C to -10 °C, and
(d2') drying of perindopril erbumine crystalline form D at temperature from 30 °C to 40 °C.

Optionally, perindopril erbumine crystalline form D obtained according to the process of the present invention may be further recrystallized from wet aliphatic ester, preferably from wet C₁-C₄ alkyl ester of C₁-C₄ aliphatic carboxylic acid or a mixture thereof.

The crystallization of perindopril erbumine from wet aliphatic ester containing from 1% (vol/vol) water to 6% (vol/vol) water according to the present invention, which preferably gives new crystalline form D, is an excellent method for removing most of diketopiperazine impurities as it is shown in Table 1.

**Table 1: Amount of diketopiperazine impurities present in perindopril erbumine prepared according to the Example 2**

| | Crude perindopril | Perindopril erbumine form D after 1 st crystallization | Perindopril erbumine form D after 2nd crystallization |
|---|---|---|---|
| Area % | 96.52 % | 99.61 % | 99.84 % |
| Diketopiperazine I | 2.33 % | 0.14 % | 0.06% |
| Diketopiperazine II | 0.54 % | 0.03 % | 0.01 % |

Another object of the present invention is related to a method of purifying perindopril erbumine comprising thermal recrystallization of perindopril erbumine from wet aliphatic ester or a mixture of wet aliphatic esters containing from 1% (vol/vol) to 6% (vol/vol) of water, more preferably containing from 2% (vol/vol) to 4% (vol/vol).

Wet aliphatic ester used for thermal recrystallization of perindopril erbumine is preferably selected from the group consisting of wet C₁-C₄ alkyl esters of C₁-C₄ aliphatic carboxylic acids. Preferably wet C₁-C₄ alkyl esters of C₁-C₄ aliphatic carboxylic acids include, wet ethyl acetate, wet isopropyl acetate, wet butyl acetate and wet ethyl propionate. More preferably the wet aliphatic ester used for thermal recrystallization of perindopril erbumine is wet ethyl acetate.

A preferred method of purifying perindopril erbumine comprises the step of:
(ai) dissolving perindopril erbumine in wet aliphatic ester or in a mixture of wet aliphatic esters containing from 1% (vol/vol) to 6% (vol/vol) of water
(bi) optionally removing of insoluble impurities by filtration,
(ci) heating the obtained mixture up to the boiling point of the used aliphatic ester or the mixture of aliphatic esters,
(di) optionally filtration of the obtained boiling solution,
(ei) cooling the obtained filtrate below 40 °C, preferably to a temperature from -10 °C to 0 °C,
(fi) isolation of perindopril erbumine crystalline form D obtained from step (ei) by filtration or centrifugation, preferably by filtration at temperature from -20 °C to -10 °C, and
(gi) drying of perindopril erbumine crystalline form D, preferably at temperature from 30 °C to 40 °C.

The crystalline perindopril erbumine, preferably, perindopril erbumine crystalline form D, as obtained after step (d), (d2), (d2') or (gi) could be formulated into a pharmaceutically acceptable dosage form, in particular wherein said dosage form is a tablet, pill, capsule or injectable.

The following examples illustrate the invention

Figure 1 represents X-ray diffraction diagram of perindopril erbumine crystalline form D obtained according to the process of the present invention.

### Example 1

### Preparation of crude perindopril

A mixture of 9.54 g of (2S,3aS,7aS)-2-carboxyperhydroindole benzyl ester, 7.26 g of N-((S)-1-carbetoxybutyl)-(S)-alanine and 12.7 g of O-(benzotriazol-1-yl)-N,N,N',N'-tetramethyluronium hexafluorophosphate in 225 ml of acetonitrile is stirred at room temperature for 30 min, then 560 ml of brine is added. The product is extracted twice with 400 ml of ethyl acetate, combined extracts are washed first with 800 ml of water, acidified with concentrated hydrochloric acid and then with 1.5 l of water. Organic phase is dried over anhydrous sodium sulphate and evaporated at 40 °C in vacuo to yield 13.5g (88%) of benzyl (2S,3aS,7aS)-((2-(1-(ethoxycarbonyl)-(S)-butylamino)-(S)-propionyl)octahydroindole-2-carboxylate (benzyl ester of perindopril).

Crude benzyl ester of perindopril (13.5 g) is dissolved in 300 ml of methanol. To the solution is added 1.35 g of catalyst (10% palladium on charcoal). The mixture is stirred at room temperature under moderate flow of hydrogen for further 5 hours. The catalyst is then filtered off, washed with 50 ml of methanol and the solution is evaporated at 50°C in vacuum. The obtained residue is crude perindopril as a clear colourless oily compound (2.33 % of diketopiperazine I, 0.54 % of diketopiperazine II).

### Example 2

### Preparation of perindopril erbumine from perindopril

Perindopril obtained from example 1 (4.0 g, 2.33 % of diketopiperazine I, 0.54 % of diketopiperazine II) is dissolved in wet ethyl acetate (40 ml, water content 4 % (vol/vol)). Insoluble impurities are filtered off, to the filtrate tert-butylamine (1.5 ml) is added under stirring at room temperature and the mixture is heated to reflux. The boiling solution is filtered and cooled to 0 °C. The product is precipitated and after 30 minutes it is filtered and dried in vacuo at 40 °C for 24 hours to obtain perindopril erbumine in crystalline form D (2.5 g, 0.14 % of diketopiperazine I, 0.03 % of diketopiperazine II).

### Example 3

### Preparation of saturated wet ethyl acetate

Ethyl acetate (100 ml) is shaken with 10 ml of water and water phase is removed. Ethyl acetate is cooled to -10 °C and pumped to the other vessel avoiding water adhered to the walls and allowed to warm to room temperature.

### Preparation of perindopril erbumine from perindopril

Perindopril obtained from example 1 (4.0 g) is dissolved in ethyl acetate (40 ml), prepared as above. Insoluble impurities are filtered off, to the filtrate tert-butylamine (1.5 ml) is added under stirring at room temperature and the mixture is heated to reflux. The boiling solution is filtered and cooled to 0 °C. The product is precipitated and after 30 minutes it is filtered and dried in vacuo at 40 °C for 24 hours to obtain perindopril erbumine in crystalline form D (2.9 g).

### Example 4

### Preparation of perindoprile erbumine form D from perindopril erbumine form α

The mixture of perindopril erbumine (5 g) and wet ethyl acetate (30 ml), prepared as in Example 3, is heated to reflux under stirring. The solution is optionally filtered and cooled to 0 °C. The product is precipitated. After 30 minutes the obtained suspension is filtered and the precipitate is dried in vacuo at 40 °C for 24 hours to yield perindopril erbumine crystalline form D (4.15 g).

### Example 5

### Preparation of perindoprile erbumine form D from perindopril erbumine form α

The mixture of perindopril erbumine (5 g) and wet isopropyl acetate (prepared from 30 ml of isopropyl acetate and 1 ml of water) is heated to reflux under stirring. The solution is optionally filtered and cooled to -10 °C, when upon it is left for 1 hour at -10 °C without agitation. The obtained suspension is filtered and the precipitate is dried in vacuo at 40 °C for 24 hours to yield perindopril erbumine crystalline form D.

Analytical data in examples are achieved using the following hardware:
Powder X-ray diffraction spectra of the sample is recorded on Siemens D-5000 with reflexion technique: CuKα radiation, range from 2° to 37° 2θ, step 0.04° 2θ, integration time 1 sec.

Chromatographic conditions for diketopiperazines determination:
1. Mobile phase:
A: dissolve 0.92 g of sodium heptanesulphonate in 1000 ml of water, add 1 ml of triethylamine and adjust to pH 2.0 with a mixture of perchloric acid and water
B: acetonitrile

2. Column: C8, 4 µm, pore size of 6 nm, 250 x 4.0 mm (Merck Supersphere 60 RP-8)
3. Conditions: temperature: 70 °C, flow rate: 1.5 ml/min, wavelength: 215 nm, injection volume: 20 µl, gradient table:

| t | % A | % B |
|---|---|---|
| 0 | 73 | 27 |
| 8 | 73 | 27 |
| 25 | 40 | 60 |
| 30 | 40 | 60 |
| 40 | 20 | 80 |
| 45 | 0 | 100 |
| 50 | 73 | 27 |

4. Relative retention with reference to perindopril (about 11 min):
diketopiperazine II - 0.56
diketopiperazine I - 1.7

5. Equipment: Waters Alliance 2695 separations module, detector PDA 2996, software Empower 5.0

## Claims

1. A process for the preparation of crystalline perindopril erbumine comprising the steps of:
(a) providing a solution of crude perindopril in wet aliphatic ester containing from 1 % (vol/vol) to 6% (vol/vol) of water or in a mixture of wet aliphatic esters containing from 1% (vol/vol) to 6% (vol/vol) of water,
(b) adding tert-butylamine to the said solution,
(c) crystallizing perindopril erbumine, and
(d) isolating crystalline perindopril erbumine.

2. A process according to claim 1, wherein said wet aliphatic ester is selected from the group consisting of wet C₁-C₄ alkyl esters of C₁-C₄ aliphatic carboxylic acids.

3. A process according to claim 1 or claim 2, wherein said wet aliphatic ester is wet ethyl acetate.

4. A process according to claim 3, wherein said wet ethyl acetate contains from 2% (vol/vol) to 4% (vol/vol) of water.

5. A process according to claim 3, wherein said wet ethyl acetate is prepared by saturation with water at temperature from -20 °C to -10 °C.

6. A process according to any one of claims 1 to 5, wherein in step (b) tert-butylamine is added at temperature from 20 °C to 40 °C.

7. A process according to any one of claims 1 to 6, wherein step (c) comprises the sub-steps of:
(c1) heating the mixture obtained from step (b) up to the boiling point of the used aliphatic ester or the mixture of aliphatic esters,
(c2) filtration of the obtained boiling solution, and
(c3) cooling the obtained filtrate below 40 °C to obtain crystalline perindopril erbumine.

8. A process according to claim 7, wherein in sub-step (c3) said filtrate in cooled to the temperature from -10 °C to 0 °C.

9. A process according to any one of claims 1 to 8, wherein step (d) comprises the sub-steps of:
(d1) isolation of crystalline perindopril erbumine obtained from step (c) by filtration or centrifugation, and
(d2) drying of crystalline perindopril erbumine.

10. A process according to claim 9, wherein said filtration in sub-step (d1) is performed at temperature below 0 °C.

11. A process according to claim 9 or claim 10, wherein said filtration in sub-step (d1) is performed at temperature from -20 °C to -10 °C.

12. A process according to any one of claims 1 to 11, wherein said crystalline perindopril erbumine obtained from step (d) contains less than about 0.10% (w/w) of diketopiperazine impurities.

13. A process according to any one of claims 1 to 12, wherein said crystalline perindopril erbumine is perindopril erbumine crystalline form D.

14. A process according to claim 13, wherein said perindopril erbumine crystalline form D has a powder x-ray diffraction pattern comprising the following characteristic reflection angles 2θ: 5.3±0.2°, 10.7±0.2°, 16.0±0.2°, 24.4±0.2° and 26.9±0.2°.

15. A process according to claim 13 or claim 14, wherein said perindopril erbumine crystalline form D has a powder x-ray diffraction pattern comprising the following characteristic 2θ angles:
| Angle 2θ (°) | Relative intensity (%) |
|---|---|
| 5.3 | 4.7 |
| 8.4 | 7.0 |
| 9.4 | 34.4 |
| 10.7 | 5.0 |
| 14.7 | 15.7 |
| 15.5 | 33.3 |
| 16.0 | 100.0 |
| 16.7 | 6.6 |
| 17.7 | 9.2 |
| 18.3 | 10.2 |
| 21.1 | 22.1 |
| 21.5 | 59.3 |
| 21.7 | 25.6 |
| 23.0 | 6.0 |
| 23.5 | 9.0 |
| 24.4 | 12.7 |
| 25.7 | 6.9 |
| 26.9 | 18.1 |
| 27.3 | 6.7 |
| 28.1 | 2.8 |

16. A method of purifying perindopril erbumine comprising thermal recrystallization of perindopril erbumine from wet aliphatic ester containing from 1% (vol/vol) to 6% (vol/vol) of water or a mixture of wet aliphatic esters containing from 1% (vol/vol) to 6% (vol/vol) of water.

## Patentansprüche

1. Ein Verfahren zur Vorbereitung von kristallenem Perindopril-Erbumin, das die folgenden Schritte umfasst:
(a) Liefern einer Lösung unbearbeiteten Perindoprils in feuchtem aliphatischem Ester, die von 1 % (Vol./Vol.) bis 6% (Vol./Vol.) Wasser enthält oder in einer Mischung feuchten aliphatischen Esters, die von 1% (Vol./Vol.) bis 6% (Vol./Vol.) Wasser enthält.
(b) Beigeben von Tert-Butylamin zur besagten Lösung,
(c) Kristallisieren von Perindopril-Erbumin und
(d) Isolieren des kristallinen Perindopril-Erbumin.

2. Ein Verfahren entsprechend Anspruch 1, wobei der besagte aliphatische Ester ausgewählt wird aus der Gruppe, die aus feuchten C1-C4-Alkyl-Estern der C1-C4-aliphatischen Carboxylsäuren besteht.

3. Ein Verfahren entsprechend Anspruch 1 oder Anspruch 2, wobei es sich bei dem besagten feuchten aliphatischen Ester um feuchtes Ethylacetat handelt.

4. Ein Verfahren entsprechend Anspruch 3, wobei das besagte feuchte Ethylacetat von 2% (Vol./Vol.) bis 4% (Vol./Vol.) Wasser enthält.

5. Ein Verfahren entsprechend Anspruch 3, wobei das besagte feuchte Ethylacetat vorbereitet wird durch Sättigung mit Wasser bei einer Temperatur von -20 °C bis -10 °C.

6. Ein Verfahren entsprechend einem der Ansprüche 1 bis 5, wobei in Schritt (b) Tert-Butylamin bei einer Temperatur von 20 °C bis 40 °C beigegeben wird.

7. Ein Verfahren entsprechend einem der Ansprüche 1 bis 6, wobei Schritt (c) die folgenden Unterschritte umfasst:
(c1) Erwärmen der in Schritt (b) erhaltenen Mischung bis zum Siedepunkt des verwendeten aliphatischen Ester oder der Mischung der aliphatischen Ester,
(c2) Filtrierung der erhaltenen Kochlösung und
(c3) Abkühlen des erhaltenen Filtrats unter 40 °C, um kristallines Perindopril-Erbumin zu erhalten.

8. Ein Verfahren entsprechend Anspruch 7, wobei das besagte Filtrat aus Unterschritt (c3) abgekühlt wird auf eine Temperatur von -10 °C bis 0 °C.

9. Ein Verfahren entsprechend einem der Ansprüche 1 bis 8, wobei Schritt (d) die folgenden Unterschritte umfasst:
(d1) Isolierung des kristallinen Perindopril-Erbumin, das in Schritt (c) durch Filtrierung oder Zentrifugierung erhalten wurde, und
(d2) Trocknung des kristallinen Perindopril-Erbumin.

10. Ein Verfahren entsprechend Anspruch 9, wobei die besagte Filtrierung in Unterschritt (d1) durchgeführt wird bei einer Temperatur von unter 0 °C.

11. Ein Verfahren entsprechend Anspruch 9 oder Anspruch 10, wobei die besagte Filtrierung im Unterschritt (d1) durchgeführt wird bei einer Temperatur von -20 °C bis -10 °C.

12. Ein Verfahren entsprechend einem der Ansprüche 1 bis 11, wobei das besagte kristalline Perindopril-Erbumin, das erhalten wird in Schritt (d), weniger als ungefähr 0,10% (Wasser/Wasser) an Diketopiperazin-Unreinheiten enthält.

13. Ein Verfahren entsprechend irgendeinem der Ansprüche 1 bis 12, wobei das besagte kristalline Perindopril-Erbumin das kristalline Perindopril-Erbumin der Form D ist.

14. Ein Verfahren entsprechend Anspruch 13, wobei das besagte kristalline Perindopril-Erbumin der Form D ein Pulver-Röntgen-Beugemuster hat, das die folgenden charakteristischen Ausfallwinkel umfasst 20:5,3+-0,2, 10,7+-0,2; 16,0+-0,2°, 24,4+-0,2° und 26,9+-0,2.

15. Ein Verfahren entsprechend Anspruch 13 oder Anspruch 14, wobei das besagte kristalline Perindopril-Erbumin der Form D ein Pulver-Röntgen-Beugemuster hat, das die folgenden charakteristischen 29 Winkel umfasst.:
| Winkel 29 (*) | Relative |
|---|---|
| | Intensität (%) |
| 5,3 | 4,7 |
| 8,4 | 7,0 |
| 9,4 | 34,4 |
| 10,7 | 5,0 |
| 14,7 | 15,7 |
| 15,5 | 33,3 |
| 16,0 | 100,0 |
| 16,7 | 6,6 |
| 17,7 | 9,2 |
| 18,3 | 10,2 |
| 21,1 | 22,1 |
| 21,5 | 59,3 |
| 21,7 | 25,6 |
| 23,0 | 6,0 |
| 23,5 | 9,0 |
| 24,4 | 12,7 |
| 25,7 | 6,9 |
| 26,9 | 18,1 |
| 27,3 | 6,7 |
| 28,1 | 2,8 |

16. Eine Methode der Reinigung von Perindopril-Erbumin, das die thermische Rekristallisierung des Perindopril-Erbumin von feuchtem aliphatischem Ester, das von 1% (vol./vol.) bis 6% (Vol./Vol.) Wasser enthält oder einer Mischung des feuchten aliphatischen Ester, das von 1 % (Vol./Vol.) bis 6% (Vol./Vol.) Wasser enthält, umfasst.

## Revendications

1. Procédé de préparation de périndopril erbumine sous forme cristalline comprenant les étapes consistant à :
(a) se procurer une solution de périndopril brut dans un ester aliphatique humide contenant de 1 % (v/v) à 6 % (v/v) d'eau ou dans un mélange d'esters aliphatiques humides contenant de 1 % (v/v) à 6 % (v/v) d'eau,
(b) ajouter de la tert-butylamine à ladite solution,
(c) laisser cristalliser la périndopril erbumine, et
(d) isoler la périndopril erbumine sous forme cristalline.

2. Procédé selon la revendication 1, dans lequel ledit ester aliphatique humide est choisi dans le groupe constitué des esters alkyliques en C₁-C₄ d'acides carboxyliques aliphatiques en C₁-C₄ humides.

3. Procédé selon la revendication 1 ou la revendication 2, dans lequel ledit ester aliphatique humide est l'acétate d'éthyle humide.

4. Procédé selon la revendication 3, dans lequel ledit acétate d'éthyle humide contient de 2 % (v/v) à 4 % (v/v) d'eau.

5. Procédé selon la revendication 3, dans lequel ledit acétate d'éthyle humide est préparé par saturation avec de l'eau à une température de -20 °C à -10 °C.

6. Procédé selon l'une quelconque des revendications 1 à 5, dans lequel dans l'étape (b) la tert-butylamine est ajoutée à une température de 20 °C à 40 °C.

7. Procédé selon l'une quelconque des revendications 1 à 6, dans lequel l'étape (c) comprend les sous-étapes de :
(c1) chauffage du mélange obtenu à l'étape (b) jusqu'au point d'ébullition de l'ester aliphatique ou du mélange d'esters aliphatiques utilisé,
(c2) filtration de la solution bouillante ainsi obtenue, et
(c3) refroidissement du filtrat ainsi obtenu en dessous de 40 °C pour obtenir de la périndopril erbumine sous forme cristalline.

8. Procédé selon la revendication 7, dans lequel dans la sous-étape (c3) ledit filtrat est refroidi à la température de -10 °C à 0 °C.

9. Procédé selon l'une quelconque des revendications 1 à 8, dans lequel l'étape (d) comprend les sous-étapes de :
(d1) isolement de la périndopril erbumine cristalline obtenue à l'étape (c), par filtration ou centrifugation, et
(d2) séchage de la périndopril erbumine cristalline.

10. Procédé selon la revendication 9, dans lequel ladite filtration dans la sous-étape (d1) est effectuée à une température inférieure à 0 °C.

11. Procédé selon la revendication 9 ou la revendication 10, dans lequel ladite filtration dans la sous-étape (d1) est effectuée à une température de -20 °C à -10 °C.

12. Procédé selon l'une quelconque des revendications 1 à 11, dans lequel ladite périndopril erbumine sous forme cristalline obtenue à l'étape (d) contient moins d'environ 0,10 % (p/p) d'impuretés de type dicétopipérazine.

13. Procédé selon l'une quelconque des revendications 1 à 12, dans lequel ladite périndopril erbumine cristalline est la forme D cristalline de la périndopril erbumine.

14. Procédé selon la revendication 13, dans lequel ladite forme D cristalline de la périndopril erbumine a un profil de diffraction des rayons X sur poudre comprenant les angles de réflexion 2θ caractéristiques suivants : 5,3 ± 0,2°, 10,7 ± 0,2°, 16,0 ± 0,2°, 24,4 ± 0,2° et 26,9 ± 0,2°.

15. Procédé selon la revendication 13 ou la revendication 14, dans lequel ladite forme D cristalline de la périndopril erbumine a un profil de diffraction des rayons X sur poudre comprenant les angles 2θ caractéristiques suivants :
| Angle 2θ (°) | Intensité relative (%) |
|---|---|
| 5,3 | 4,7 |
| 8,4 | 7,0 |
| 9,4 | 34,4 |
| 10,7 | 5,0 |
| 14,7 | 15,7 |
| 15,5 | 33,3 |
| 16,0 | 100,0 |
| 16,7 | 6,6 |
| 17,7 | 9,2 |
| 18,3 | 10,2 |
| 21,1 | 22,1 |
| 21,5 | 59,3 |
| 21,7 | 25,6 |
| 23,0 | 6,0 |
| 23,5 | 9,0 |
| 24,4 | 12,7 |
| 25,7 | 6,9 |
| 26,9 | 18,1 |
| 27,3 | 6,7 |
| 28,1 | 2,8 |

16. Procédé de purification de la périndopril erbumine comprenant la recristallisation thermique de la périndopril erbumine dans un ester aliphatique humide contenant de 1 % (v/v) à 6 % (v/v) d'eau ou dans un mélange d'esters aliphatiques humides contenant de 1 % (v/v) à 6 % (v/v) d'eau.
